(19)

Europäisches
Patentamt
European
Patent Office
Office européen
des brevets

(11) **EP 4 644 893 A1**

(12) **EUROPEAN PATENT APPLICATION**

(43) Date of publication:
**05.11.2025 Bulletin 2025/45**

(21) Application number: **25167796.9**

(22) Date of filing: **01.04.2025**

(51) International Patent Classification (IPC):
**G01N 33/00** (2006.01)

(52) Cooperative Patent Classification (CPC):
**G01N 33/0062**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **30.04.2024 US 202418650261**

(71) Applicant: **Rebellion Photonics, Inc.
Houston, TX 77002 (US)**

(72) Inventors:
- **SHEN, Quan
  Charlotte, 28202 (US)**
- **AN, Lingling
  Charlotte, 28202 (US)**

(74) Representative: **Houghton, Mark Phillip
Patent Outsourcing Limited
1 King Street
Bakewell, Derbyshire DE45 1DZ (GB)**

(54) **SYSTEMS, APPARATUSES, METHODS, AND COMPUTER PROGRAM PRODUCTS FOR PERFORMING GAS ANALYSIS**

(57)      Systems, apparatuses, methods, and computer program products for performing gas analysis are provided. An example gas analysis system may comprise at least one gas detection sensor and at least one controller component. In some embodiments, the controller component is configured to obtain image data of a target area. In some embodiments, the controller component is configured to generate, by applying the image data to a gas plume impact model, gas plume impact data. In some embodiments, the controller component is configured to generate refined gas quantity data based at least in part on the gas plume impact data. In some embodiments, the controller component is configured to initiate performance of one or more responsive actions based at least in part on the refined gas quantity data.

FIG. 1

EP 4 644 893 A1

**Description**

**FIELD**

Embodiments of the present disclosure relate generally to systems, apparatuses, methods, and computer program products for performing gas analysis.

BACKGROUND

Applicant has identified many technical challenges and difficulties associated with systems, apparatuses, methods, and computer program products for performing gas analysis. Through applied effort, ingenuity, and innovation, Applicant has solved problems related to systems, apparatuses, methods, and computer program products for performing gas analysis by developing solutions embodied in the present disclosure, which are described in detail below.

BRIEF SUMMARY

Various embodiments described herein relate to systems, apparatuses, methods, and computer program products for performing gas analysis.

In accordance with one aspect of the disclosure, a gas analysis system is provided. In some embodiments, the gas analysis system may include at least one gas detection sensor. In some embodiments, the gas analysis system may include a controller component. In some embodiments, the controller component is configured to obtain image data of a target area. In some embodiments, the image data comprises a series of image frames. In some embodiments, a first portion of the series of image frames are indicative of a gas plume. In some embodiments, the controller component is configured to generate, by applying the image data to a gas plume impact model, gas plume impact data. In some embodiments, the controller component is configured to generate refined gas quantity data based at least in part on the gas plume impact data. In some embodiments, the controller component is configured to initiate performance of one or more responsive actions based at least in part on the refined gas quantity data.

In some embodiments, the target area is associated with an asset.

In some embodiments, the asset is a processing plant.

In some embodiments, the controller component is further configured to perform a moving average operation on the refined gas quantity data.

In some embodiments, a second portion of the series of image frames are not indicative of the gas plume.

In some embodiments, applying the image data to the gas plume impact model to generate gas plume impact data comprises the gas plume impact model being configured to identify a first image frame in the second portion of the series of image frames.

In some embodiments, the first image frame is immediately preceded in the series of image frames by a second image frame in the first portion of the series of image frames.

In some embodiments, applying the image data to the gas plume impact model to generate gas plume impact data comprises the gas plume impact model being configured to generate zero hit count data based at least in part on the first image frame being immediately preceded by the second image frame in the series of image frames.

In some embodiments, applying the image data to the gas plume impact model to generate gas plume impact data comprises the gas plume impact model being configured to identify a first image frame in the second portion of the series of image frames.

In some embodiments, the first image frame is immediately followed in the series of image frames by a second image frame in the first portion of the series of image frames.

In some embodiments, applying the image data to the gas plume impact model to generate gas plume impact data comprises the gas plume impact model being configured to generate zero hit count data based at least in part on the first image frame being immediately followed by the second image frame in the series of image frames.

In some embodiments, generating the refined gas quantity data comprises generating, by applying the gas plume impact data to raw gas quantity data, a first portion of the refined gas quantity data.

In some embodiments, the raw gas quantity data is generated by applying the image data to a gas quantity determination machine learning model.

In some embodiments, generating the refined gas quantity data comprises generating, by performing an interpolation operation on the first portion of the refined gas quantity data, a second portion of the refined gas quantity data.

In accordance with another aspect of the disclosure, a method is provided. In some embodiments, the method may include obtaining image data of a target area. In some embodiments, the image data comprises a series of image frames. In some embodiments, a first portion of the series of image frames are indicative of a gas plume. In some embodiments, the method may include generating, by applying the image data to a gas plume impact model, gas plume impact data. In some embodiments, the method may include generating refined gas quantity data based at least in part on the gas plume impact data. In some embodiments, the method may include initiating performance of one or more responsive actions based at least in part on the refined gas quantity data.

In accordance with another aspect of the disclosure, a computer program product is provided. In some embodiments, the computer program product includes at least one non-transitory computer-readable storage medium having computer program code stored thereon. In some embodiments, the computer program code, in execution with at least one processor, configures the computer program product for obtaining image data of a target area. In some embodiments, the image data comprises a series of image frames. In some embodiments, a first portion of the series of image frames are indicative of a gas plume. In some embodiments, the computer program code, in execution with at least one processor, configures the computer program product for generating, by applying the image data to a gas plume impact model, gas plume impact data. In some embodiments, the computer program code, in execution with at least one processor, configures the computer program product for generating refined gas quantity data based at least in part on the gas plume impact data. In some embodiments, the computer program code, in execution with at least one processor, configures the computer program product for initiating performance of one or more responsive actions based at least in part on the refined gas quantity data.

## BRIEF DESCRIPTION OF THE DRAWINGS

The description of the illustrative examples may be read in conjunction with the accompanying figures. It will be appreciated that, for simplicity and clarity of illustration, components and elements illustrated in the figures have not necessarily been drawn to scale, unless described otherwise. For example, the dimensions of some of the components or elements may be exaggerated relative to other elements, unless described otherwise. Examples incorporating teachings of the present disclosure are shown and described with respect to the figures presented herein, in which:

FIG. 1 illustrates an example schematic diagram depicting an environment in accordance with various examples of the present disclosure;
FIG. 2 illustrates a schematic diagram depicting an example system in accordance with various examples of the present disclosure;
FIG. 3 illustrates a perspective view of an example gas analysis system in accordance with various examples of the present disclosure;
FIG. 4 illustrates an example controller component of a gas analysis system in accordance with various embodiments of the present disclosure;
FIG. 5 illustrates an example series of image frames in accordance various embodiments of the present disclosure;

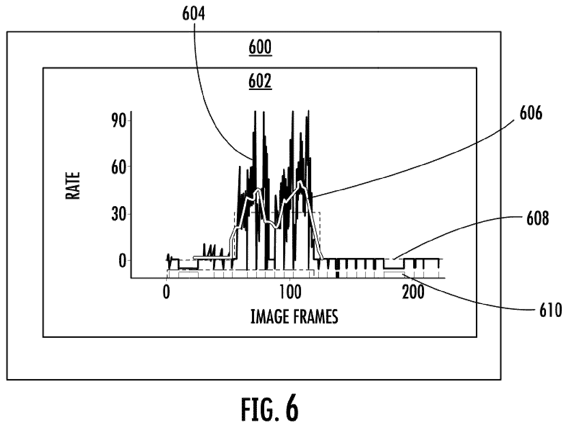

FIG. 6 illustrates an example interface component in accordance various embodiments of the present disclosure;

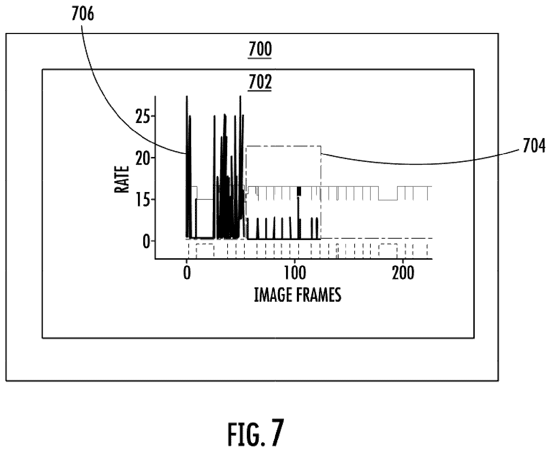

FIG. 7 illustrates another example interface component in accordance various embodiments of the present disclosure;
FIG. 8 illustrates a flowchart of an example method in accordance with one or more embodiments of the present disclosure;
FIG. 9 illustrates a flowchart of another example method in accordance with one or more embodiments of the present disclosure;
FIG. 10 illustrates a flowchart of another example method in accordance with one or more embodiments of the present disclosure; and
FIG. 11 illustrates a flowchart of another example method in accordance with one or more embodiments of the present disclosure.

## DETAILED DESCRIPTION OF THE INVENTION

Some examples of the present disclosure will now be described more fully hereinafter with reference to the accompanying drawings, in which some, but not all examples of the disclosure are shown. Indeed, these disclosures may be embodied in many different forms and should not be construed as limited to the examples set forth herein; rather, these examples are provided so that this disclosure will satisfy applicable legal requirements. Like numbers refer to like elements throughout.

The phrases "in one example," "according to one example," "in some examples," and the like generally mean that the particular feature, structure, or characteristic following the phrase may be included in at least one example of the present disclosure and may be included in more than one example of the present disclosure (importantly, such phrases do not necessarily refer to the same example).

If the specification states a component or feature "may," "can," "could," "should," "would," "preferably," "possibly," "typically," "optionally," "for example," "as an example," "in some examples," "often," or "might" (or other such language) be included or have a characteristic, that specific component or feature is not required to be included or to have the characteristic. Such component or feature may be optionally included in some examples, or it may be excluded.

The word "example" or "exemplary" is used herein to mean "serving as an example, instance, or illustration." Any implementation described herein as "exemplary" is not necessarily to be construed as preferred or advantageous over other implementations.

The term "electronically coupled," "electronically coupling," "electronically couple," "in communication with," "in electronic communication with," or "connected" in the present disclosure refers to two or more elements or components being connected through wired means and/or wireless means, such that signals, electrical voltage/current, data and/or information may be transmitted to and/or received from these elements or components.

Overview

Example embodiments disclosed herein address technical problems associated with systems, apparatuses, methods, and computer program products for performing gas analysis. As would be understood by one skilled in the field to which this disclosure pertains, there are numerous example scenarios in which a user may use systems, apparatuses, methods, and computer program products for performing gas analysis.

In many applications, systems, apparatuses, methods, and computer program products for performing gas analysis are desirable. In some examples, it may be desirable to perform gas analysis for an asset, such as a processing plant. In some examples, it may be desirable to perform a gas analysis for multiple target areas (e.g., locations) of an asset. In some examples, it may be desirable to perform a gas analysis to quantify the amount of gas released by a gas plume associated with an asset based on one or more properties of the gas plume that are identified and analyzed using image data. For example, it may be desirable to quantify the amount of gas released by a gas plume associated with an asset based on the shape, size, and/or concentration of a gas plume. In some examples, a gas plume may be associated with a gas leak at an asset. In this way, it may be desirable to identify and remedy gas leaks at an asset as well as quantify the amount of gas leaked into an environment associated with the asset to ensure that the asset is adhering to its goals for reducing greenhouse gas emissions.

Example solutions for performing a gas analysis for an asset include identifying and monitoring gas plumes to quantify an amount of gas associated with a gas plume (e.g., a gas plume associated with a gas leak). In some examples, one or more properties of a gas plume (e.g., gas plume size, gas plume shape, gas plume concentration) may be impacted by conditions associated with the asset. For example, conditions such as wind, humidity, and/or the availability of gas detection sensors to continuously monitor a gas plume may impact one or more properties of a gas plume. In some examples, as the properties of a gas plume change, the amount of gas associated with a gas plume may change (e.g., the amount of gas leaked by a gas plume may change). However, existing example solutions do not contemplate accounting for conditions associated with the asset and/or properties of the gas plume when quantifying an amount of gas associated with a gas plume. As such, such example solutions are often unable to accurately quantify an amount of gas released by a gas plume. Additionally, in such example solutions, many cameras are needed to monitor each potential location where a gas plume may occur. As such, such example solutions are often costly and inefficient. Accordingly, there is a need for systems, apparatuses, methods, and computer program products for performing gas analysis that are able to monitor multiple target areas for gas plumes and determine the amount of gas leaked into an environment due to a gas plume in an efficient and accurate manner that accounts for conditions associated with the asset.

Thus, to address these and/or other issues related performing a gas analysis, example systems, apparatuses, methods, and computer program products for performing a gas analysis are disclosed herein. For example, an embodiment in this disclosure, described in greater detail below, includes a gas analysis system that includes at least one gas detection sensor and a controller component. In some embodiments, the controller component may be configured to obtain image data of a target area, wherein the image data comprises a series of image frames, wherein a first portion of the series of image frames are indicative of a gas plume. In some embodiments, the controller component may be configured to generate, by applying the image data to a gas plume impact model, gas plume impact data. In some embodiments, the controller component may be configured to generate refined gas quantity data based at least in part on the gas plume impact data. In some embodiments, the controller component may be configured to initiate performance of one or more responsive actions based at least in part on the refined gas quantity data. Accordingly, the systems, apparatuses, methods, and computer program products disclosed herein enable performance of a gas analysis of multiple target areas and for quantification of the amount of gas associated with a gas plume in an efficient, accurate, and cost-effective manner.

Example Systems and Apparatuses

Embodiments of the present disclosure herein include systems, apparatuses, methods, and computer program products configured for performing gas analysis. It should be readily appreciated that the embodiments of the apparatus, systems, methods, and computer program product described herein may be configured in various additional and alternative manners in addition to those expressly described herein.

Various examples of the present disclosure may provide example technical improvements on the performance of gas analysis systems. Gas analysis systems may be configured to quantify, detect, measure, and/or identify a size, shape, and/or concentration level of one or more gaseous substances in a particular area or location, such as a size, shape, and/or concentration of a gas plume in a particular area or location. In particular, gas analysis systems may be utilized in environments where there is a high risk of gas leaks that may result in fires, explosions and/or acute toxic exposure such as an asset, components of an asset, and/or the like. One example of a gas analysis system is as a hyperspectral gas analysis system. An example hyperspectral gas analysis system may comprise one or more gas detection sensors (e.g., cameras) that are configured to obtain and analyze raw image/video data (e.g., hyperspectral image data and visible image data) associated with one or more spectral bands (e.g., infrared, visible light) of the electromagnetic spectrum. In some examples, the hyperspectral gas analysis system may be configured to detect one or more gaseous substances including, but not limited to, acetic acid, Ammonia, Benzene, Butadiene, Butane, Ethane, Ethanol, Ethylene, Iso-Butylene, Iso-Pentane, Methane, Methanol, N-Pentane, Propane, Propylene, Toluene, Vinyl Chloride, p- or m-Xylene, and/or the like.

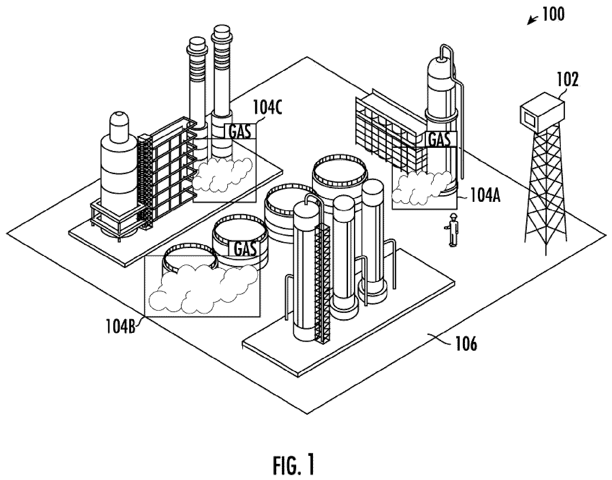

Referring now to FIG. 1, a schematic diagram depicting an environment 100 in accordance with various embodiments of the present disclosure is provided. In some embodiments, the environment 100 may include an asset 106. In some embodiments, for example, the asset 106 may be any type of plant associated with the environment 100. In this regard, the asset 106 may, for example, be a processing plant that receives and processes ingredients as inputs to create a processed product, such as a hydrocarbon processing plant, a refinery, a pulp and paper plant, a chemical plant, an alumina plant, a drilling facility, a fracking field, an oil drilling rig, an offshore platform, a gas sale station, a liquified natural gas vessel, a gas production facility, a gas transmission vehicle, a gas station, and/or the like. Additionally, or alternatively, for example, the asset 106 may include at least one building. In this regard, the asset 106 may, for example, be an industrial building, office building, building associated with a plant, and/or the like.

The asset 106 in some embodiments includes any number of individual components. The components of the asset 106 may perform a particular function during operation of the asset 106. For example, the components may include one or more well components, fracking components, crude processing components, hydrotreating components, isomerization components, vapor recovery components, fluid catalytic cracking components, hydrocracking components, aromatics reduction components, visbreaker components, storage tank components, blender components, pump components, flash venting components, compressor components, cooler components (e.g., air cooler components), sensor components, storage components, flare components, heating, ventilation, and air (HVAC) components, lighting components, and/or the like that perform a particular operation for transforming, storing, releasing, and/or otherwise handling one or more input ingredient(s) (e.g., hydrocarbons, gases, etc.). In this regard, for example, the individual components of a plant may include components associated with a particular process performed by the plant.

In some embodiments, the environment 100 may include a gas analysis system 102. In some embodiments, the gas analysis system 102 may be configured to monitor the environment 100 and/or the asset 106. In some embodiments, the gas analysis system 102 may be positioned within the asset 106 and/or in proximity of the asset 106. In some embodiments, at least a portion of the gas analysis system 102 is moveable with respect to a fixed location such that the gas analysis system 102 can move (e.g., rotate, pan, tilt, and/or the like) to facilitate monitoring of a plurality of target areas within the environment 100 and/or asset 106. As illustrated, the example gas analysis system 102 is configured to monitor at least a first target area 104A, a second target area 104B, and a third target area 104C. In this regard, for example, at least a portion of the gas analysis system 102 may be moved (e.g., rotated, paned, titled, and/or the like) to different positions to capture image data. For example, at least a portion of the gas analysis system 102 may be moved (rotated, paned, titled, and/or the like) from a first position associated with the first target area 104A to a second position associated with the second target area 104B (e.g., at least a portion of the gas analysis system 102 may be rotated from the first position to the second position).

In various embodiments, the gas analysis system 102 is configured to generate a calibrated image/video stream using a particular light source (e.g., blackbody radiation, infrared radiation, and/or the like) in order to detect an absorption signature of one or more gaseous substances. For example, to detect an absorption signature of one or more gaseous substances in one or more of the plurality of target areas with the environment 100 and/or asset 106.

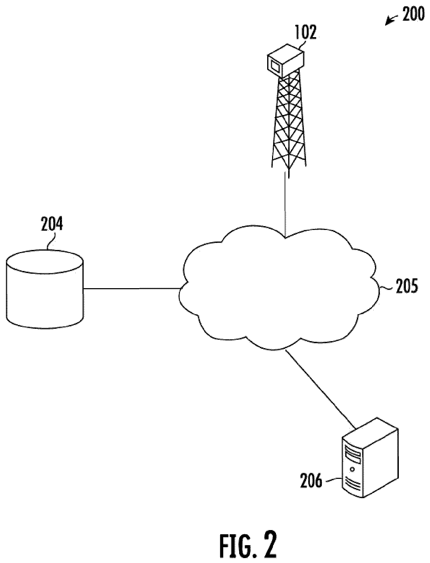

Referring now to FIG. 2, an example schematic diagram depicting an example system 200 in accordance various embodiments of the present disclosure is provided. As depicted, the example system 200 comprises the gas analysis system 102, one or more computing entities 206 (e.g., servers), one or more databases 204, one or more networks 205, and/or the like. In various examples, the system 200 may operate to facilitate monitoring of one or more gaseous substances within a particular location or environment.

In various embodiments, the gas analysis system 102 may be or comprise a hyperspectral gas analysis system that is configured to obtain image data (e.g., video streams) within a location (e.g., the environment 100). For example, the gas analysis system 102 may be configured to obtain first image data and/or second image data. In some examples, the gas analysis system 102 may capture image data at a rate of 15 images per second. As discussed above in connection to FIG. 1, the example gas analysis system 102 may be stationary (e.g., mounted on a platform, tower, support structure, and/or the like). In various embodiments, the gas analysis system 102, the one or more databases 204, and/or the one or more user computing entities 108 (e.g., servers) are in electronic communication with each other over the one or more networks 205 such that they can exchange data (e.g., receive and transmit data) with one another (e.g., periodically, and/or in response to requests). Each of the components of the system 200, including the gas analysis system 102, the one or more computing entities 206, and/or the one or more databases 204, may be in communication with one another over the same or different wireless or wired networks 205 including, for example, a wired or wireless Personal Area Network (PAN), Local Area Network (LAN), Metropolitan Area Network (MAN), Wide Area Network (WAN), cellular network, and/or the like. While FIG. 2 illustrates certain system components as separate, standalone devices, the various embodiments are not limited to this particular architecture.

As depicted in FIG. 2, the example system 200 comprises one or more computing entities 206. In general, the terms computing device, entity, device, system, and/or similar words used herein interchangeably may refer to, for example, one or more computers, computing devices, computing entities, desktop computers, mobile phones, tablets, phablets, notebooks, laptops, distributed systems, terminals, servers or server networks, blades, gateways, switches, processing devices, set-top boxes, relays, routers, network access points, base stations, the like, and/or any combination of devices adapted to perform the functions, operations, and/or processes described herein. Such functions, operations, and/or processes may include, for example, transmitting, receiving, operating on, processing, displaying, storing, determining, generating/creating, monitoring, evaluating, comparing, and/or similar terms used herein interchangeably. In one embodiment, these functions, operations, and/or processes can be performed on data, content, information, and/or similar terms used herein interchangeably.

In some examples, the computing entity 206 may also include one or more network and/or communications interfaces for communicating with various computing entities, such as by communicating data, content, information, and/or similar terms used herein interchangeably that can be transmitted, received, operated on, processed, displayed, stored, and/or the like.

In one embodiment, the computing entity 206 may further include or be in communication with non-volatile media (also referred to as non-volatile storage, memory, memory storage, memory circuitry and/or similar terms used herein interchangeably). In one embodiment, the non-volatile storage or memory may include one or more non-volatile storage or memory media as described above, such as hard disks, ROM, PROM, EPROM, EEPROM, flash memory, MMCs, SD memory cards, Memory Sticks, CBRAM, PRAM, FeRAM, RRAM, SONOS, racetrack memory, and/or the like. As will be recognized, the non-volatile storage or memory media may store databases, database instances, database management system entities, data, applications, programs, program modules, scripts, source code, object code, byte code, compiled code, interpreted code, machine code, executable instructions, and/or the like. The term database, database instance, database management system entity, and/or similar terms used herein interchangeably may refer to a structured collection of records or information/data that is stored in a computer-readable storage medium, such as via a relational database, hierarchical database, and/or network database.

In one embodiment, the computing entity 206 may further include or be in communication with volatile media (also referred to as volatile storage, memory, memory storage, memory circuitry and/or similar terms used herein interchangeably). In one embodiment, the volatile storage or memory may also include one or more volatile storage or memory media as described above, such as RAM, DRAM, SRAM, FPM DRAM, EDO DRAM, SDRAM, DDR SDRAM, DDR2 SDRAM, DDR3 SDRAM, RDRAM, RIMM, DIMM, SIMM, VRAM, cache memory, register memory, and/or the like. As will be recognized, the volatile storage or memory media may be used to store at least portions of the databases, database instances, database management system entities, data, applications, programs, program modules, scripts, source code, object code, byte code, compiled code, interpreted code, machine code, executable instructions, and/or the like being executed by, for example, the processing element. Thus, the databases, database instances, database management system entities, data, applications, programs, program modules, scripts, source code, object code, byte code, compiled code, interpreted code, machine code, executable instructions, and/or the like may be used to control certain aspects of the operation of the computing entity 206 with the assistance of the processing element and the operating system.

As indicated, in one embodiment, the computing entity 206 may also include one or more network and/or communications interfaces for communicating with various computing entities, such as by communicating data, content, information, and/or similar terms used herein interchangeably that can be transmitted, received, operated on, processed, displayed, stored, and/or the like. Such communication may be executed using a wired data transmission protocol, such as fiber distributed data interface (FDDI), digital subscriber line (DSL), Ethernet, asynchronous transfer mode (ATM), frame relay, data over cable service interface specification (DOCSIS), or any other wired transmission protocol. Similarly, computing entity 206 may be configured to communicate via wireless external communication networks using any of a variety of protocols, such as general packet radio service (GPRS), Universal Mobile Telecommunications System (UMTS), Code Division Multiple Access 200 (CDMA200), CDMA200 1X (1xRTT), Wideband Code Division Multiple Access (WCDMA), Global System for Mobile Communications (GSM), Enhanced Data rates for GSM Evolution (EDGE), Time Division-Synchronous Code Division Multiple Access (TD-SCDMA), Long Term Evolution (LTE), Evolved Universal Terrestrial Radio Access Network (E-UTRAN), Evolution-Data Optimized (EVDO), High Speed Packet Access (HSPA), High-Speed Downlink Packet Access (HSDPA), IEEE 802.11 (Wi-Fi), Wi-Fi Direct, 802.16 (WiMAX), ultra-wideband (UWB), IR protocols, NFC protocols, RFID protocols, IR protocols, ZigBee protocols, Z-Wave protocols, 6LoWPAN protocols, Wibree, Bluetooth protocols, wireless universal serial bus (USB) protocols, and/or any other wireless protocol. The computing entity 206 may use such protocols and standards to communicate using Border Gateway Protocol (BGP), Dynamic Host Configuration Protocol (DHCP), Domain Name System (DNS), File Transfer Protocol (FTP), Hypertext Transfer Protocol (HTTP), HTTP over TLS/SSL/Secure, Internet Message Access Protocol (IMAP), Network Time Protocol (NTP), Simple Mail Transfer Protocol (SMTP), Telnet, Transport Layer Security (TLS), Secure Sockets Layer (SSL), Internet Protocol (IP), Transmission Control Protocol (TCP), User Datagram Protocol (UDP), Datagram Congestion Control Protocol (DCCP), Stream Control Transmission Protocol (SCTP), HyperText Markup Language (HTML), and/or the like.

As will be appreciated, one or more of the computing entity's 206 components may be located remotely from other computing entity 206 components, such as in a distributed system. Furthermore, one or more of the components may be aggregated and additional components performing functions described herein may be included in the computing entity 206. Thus, the computing entity 206 can be adapted to accommodate a variety of needs and circumstances, such as including various components described with regard to a mobile application executing on a user computing entity, including various input/output interfaces.

As depicted in FIG. 2, any two or more of the illustrative components of the system 200 of FIG. 2 may be configured to communicate with one another via one or more networks 205. The networks 205 may include, but are not limited to, any one or a combination of different types of suitable communications networks such as, for example, cable networks, public networks (e.g., the Internet), private networks (e.g., frame-relay networks), wireless networks, cellular networks, telephone networks (e.g., a public switched telephone network), or any other suitable private and/or public networks. Further, the networks 205 may have any suitable communication range associated therewith and may include, for example, global networks (e.g., the Internet), MANs, WANs, LANs, or PANs. In addition, the networks 205 may include any type of medium over which network traffic may be carried including, but not limited to, coaxial cable, twisted-pair wire, optical fiber, a hybrid fiber coaxial (HFC) medium, microwave terrestrial transceivers, radio frequency communication mediums, satellite communication mediums, or any combination thereof, as well as a variety of network devices and computing platforms provided by network providers or other entities.

While FIG. 2 provides an example system 200, it is noted that the scope of the present disclosure is not limited to the example shown in FIG. 2. In some examples, the system 200 may comprise one or more additional and/or alternative elements, and/or may be different from that illustrated in FIG. 2.

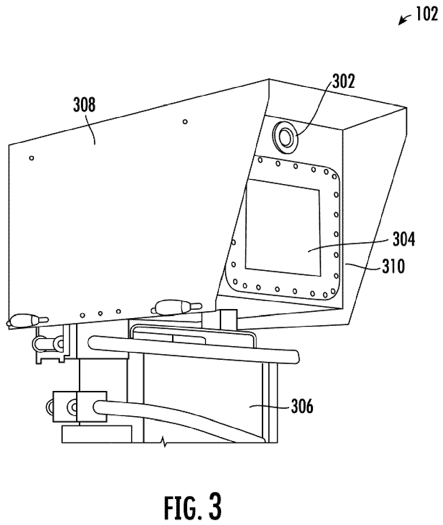

Referring now to FIG. 3, a perspective view of the gas analysis system 102 (e.g., hyperspectral gas analysis system) in accordance various embodiments of the present disclosure is provided. As depicted, the example gas analysis system 102 may comprise a housing 308 configured to contain one or more elements/components of the gas analysis system 102. For example, the housing 308 may include the controller component of the gas analysis system 102. As another example, the housing 308 may include, partially include, and/or partially cover, the at least one gas detection sensor 310 of the gas analysis system 102.

As depicted in FIG. 3, the at least one gas detection sensor 310 of the gas analysis system 102 may include at least one visible gas detection sensor 302 (e.g., an RGB camera). Additionally, or alternatively, the at least one gas detection sensor 310 of the gas analysis system 102 may include at least one hyperspectral gas detection sensor 304. In various embodiments, the gas analysis system 102 is configured to obtain, monitor, and/or capture image data (e.g., infrared image data, visible image data, combinations thereof, and/or the like) via the at least one gas detection sensor 310, such as via the at least one visible gas detection sensor 302 and the at least one hyperspectral gas detection sensor 304. For example, the gas analysis system 102 may be configured to obtain, monitor, and/or capture first image data and/or second image data via the at least one gas detection sensor 310, such as via the at least one visible gas detection sensor 302 and the at least one hyperspectral gas detection sensor 304. In various examples, the gas analysis system 102 is be configured to generate a calibrated image using a particular light source (e.g., blackbody radiation, infrared radiation, and/or the like) in order to detect an absorption signature of one or more gaseous substances, such as an absorption signature of a gas plume.

As further depicted in FIG. 3, the gas analysis system 102 may comprise a pan-tilt unit 306 that operates to enable movement (e.g., rotations, pans, tilts, and/or the like) of at least a portion of the gas analysis system 102 (e.g., in some examples, 360 degree rotations and/or up to a 45 degree tilt) to facilitate monitoring of more than one target area within a particular location. For example, the housing 308 and/or the at least one gas detection sensor 310 may be moved by the pan-tilt unit 306 to capture first image data from the first target area 104A and second image data from the second target area 104B (e.g., may be moved from a first position associated with the first target area 104A to a second position associated with the second target area 104B). In some embodiments, the example gas analysis system 102 may be mounted on a fixed/stationary support structure (e.g., tower, base, frame, interior building surface, and/or the like) within the environment 100 and/or the asset 106.

While FIG. 3 provides an example gas analysis system 102, it is noted that the scope of the present disclosure is not limited to the example shown in FIG. 3. In some examples, the gas analysis system 102 may comprise one or more additional and/or alternative elements, and/or may be different from that illustrated in FIG. 3. For example, the at least one gas detection sensor 310 may include one or more of an electrochemical sensing component, a catalytic sensing component, and/or a photoionization sensing component.

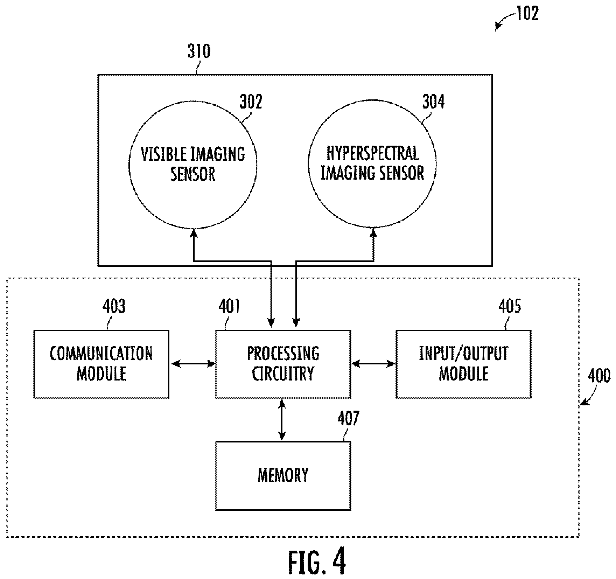

Referring now to FIG. 4, a schematic diagram depicting an example controller component 400 of the gas analysis system 102 in electronic communication with various other components in accordance with various embodiments of the present disclosure. As shown, the controller component 400 comprises processing circuitry 401, a communication module 403, input/output module 405, a memory 407, and/or other components configured to perform various operations, procedures, functions or the like described herein.

As shown, the controller component 400 (such as the processing circuitry 401, communication module 403, input/output module 405 and memory 407) is electrically coupled to and/or in electronic communication with at least the at least one gas detection sensor 310, the visible gas detection sensor 302, and/or the hyperspectral gas detection sensor 304. As depicted, the at least one gas detection sensor, the visible gas detection sensor 302, and/or the hyperspectral gas detection sensor 304 may exchange (e.g., transmit and receive) data with the processing circuitry 401 of the controller component 400.

The processing circuitry 401 may be implemented as, for example, various devices comprising one or a plurality of microprocessors with accompanying digital signal processors; one or a plurality of processors without accompanying digital signal processors; one or a plurality of coprocessors; one or a plurality of multi-core processors; one or a plurality of controllers; processing circuits; one or a plurality of computers; and various other processing elements (including integrated circuits, such as ASICs or FPGAs, or a certain combination thereof). In some embodiments, the processing circuitry 401 may comprise one or more processors. In one exemplary embodiment, the processing circuitry 401 is configured to execute instructions stored in the memory 407 or otherwise accessible by the processing circuitry 401. When executed by the processing circuitry 401, these instructions may enable the controller component 400 to execute one or a plurality of the functions as described herein. No matter whether it is configured by hardware, firmware/software methods, or a combination thereof, the processing circuitry 401 may comprise entities capable of executing operations according to the embodiments of the present invention when correspondingly configured. Therefore, for example, when the processing circuitry 401 is implemented as an ASIC, an FPGA, or the like, the processing circuitry 401 may comprise specially configured hardware for implementing one or a plurality of operations described herein. Alternatively, as another example, when the processing circuitry 401 is implemented as an actuator of instructions (such as those that may be stored in the memory 407), the instructions may specifically configure the processing circuitry 401 to execute one or a plurality of algorithms and operations described herein, such as those discussed with reference to FIG. 4.

The memory 407 may comprise, for example, a volatile memory, a non-volatile memory, or a certain combination thereof. Although illustrated as a single memory in FIG. 4, the memory 407 may comprise a plurality of memory components. In various embodiments, the memory 407 may comprise, for example, a hard disk drive, a random-access memory, a cache memory, a flash memory, a Compact Disc Read-Only Memory (CD-ROM), a Digital Versatile Disk Read-Only Memory (DVD-ROM), an optical disk, a circuit configured to store information, or a certain combination thereof. The memory 407 may be configured to store information, data, application programs, instructions, and etc., so that the controller component 400 can execute various functions according to the embodiments of the present disclosure. For example, in at least some embodiments, the memory 407 is configured to cache input data for processing by the processing circuitry 401. Additionally, or alternatively, in at least some embodiments, the memory 407 is configured to store program instructions for execution by the processing circuitry 401. The memory 407 may store information in the form of static and/or dynamic information. When the functions are executed, the stored information may be stored and/or used by the controller component 400.

The communication module 403 may be implemented as any apparatus included in a circuit, hardware, a computer program product or a combination thereof, which is configured to receive and/or transmit data from/to another component or apparatus. The computer program product comprises computer-readable program instructions stored on a computer-readable medium (for example, the memory 407) and executed by a controller component 400 (for example, the processing circuitry 401). In some embodiments, the communication module 403 (as with other components discussed herein) may be at least partially implemented as the processing circuitry 401 or otherwise controlled by the processing circuitry 401. In this regard, the communication module 403 may communicate with the processing circuitry 401, for example, through a bus. The communication module 403 may comprise, for example, antennas, transmitters, receivers, transceivers, network interface cards and/or supporting hardware and/or firmware/software and is used for establishing communication with another apparatus. The communication module 403 may be configured to receive and/or transmit any data that may be stored by the memory 407 by using any protocol that can be used for communication between apparatuses. The communication module 403 may additionally or alternatively communicate with the memory 407, the input/output module 405 and/or any other component of the controller component 400, for example, through a bus.

In some embodiments, the controller component 400 may comprise an input/output module 405. The input/output module 405 may communicate with the processing circuitry 401 to receive instructions input by the user and/or to provide audible, visual, mechanical or other outputs to the user. Therefore, the input/output module 405 may comprise supporting devices, such as a keyboard, a mouse, a display, a touch screen display, and/or other input/output mechanisms. Alternatively, at least some aspects of the input/output module 405 may be implemented on a device used by the user to communicate with the controller component 400. The input/output module 405 may communicate with the memory 407, the communication module 403 and/or any other component, for example, through a bus. One or a plurality of input/output modules and/or other components may be included in the controller component 400.

In some embodiments, the controller component 400 of the gas analysis system 102 may be configured to obtain image data. In some embodiments, image data may be one or more items of data indicative of and/or associated with images of a target area. For example, image data may include first image data associated with the first target area 104A and/or second image data associated with the second target area 104B. In some embodiments, image data may include hyperspectral image data. In some embodiments, hyperspectral image data may be image data that is associated with a plurality of bands across the electromagnetic spectrum (e.g., infrared, visible light spectrum, x-rays, ultraviolet, combinations thereof, and/or the like). Additionally, or alternatively, image data may include visible image data. In some embodiments, visible image data may be image data that is associated with the visible light bank of the electromagnetic spectrum.

In some embodiments, image data may be used to identify one or more properties associated with a gas plume in the image data. For example, image data may be used to identify one or more of a size associated with a gas plume, a shape associated with a gas plume, a concentration associated with a gas plume, and/or the like. In this way, an amount of gas associated with a gas plume in the image data may be quantified. For example, if a gas plume in the image data is associated with a gas leak, the image data may be used to quantify an amount of gas that is leaked into the environment 100 and/or the asset 106 due to the gas leak.

In some embodiments, the controller component 400 may be configured to obtain image data from the at least one gas detection sensor 310. For example, the controller component 400 may be configured to obtain first image data of the first target area 104A from the at least one gas detection sensor 310. As another example, the controller component 400 may be configured to obtain second image data of the second target area 104B from the at least one gas detection sensor 310. In some embodiments, the controller component 400 may be configured to cause the at least one gas detection sensor 310 to capture image data (e.g., using the visible gas detection sensor 302 and/or the hyperspectral gas detection sensor 304).

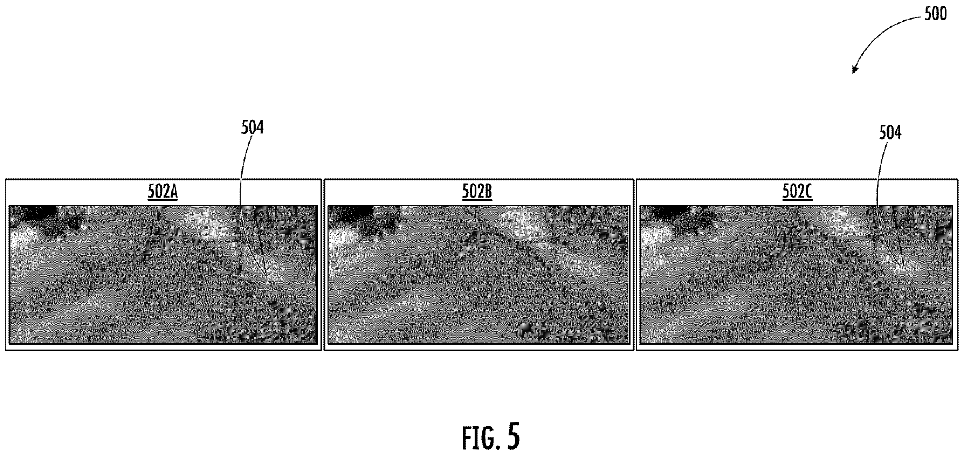

In some embodiments, image data may include a series of image frames 500. For example, the series of image frames 500 may include a first image frame 502A. As another example, the series of image frames 500 may include a second image frame 502B. As another example, the series of image frames 500 may include a third image frame 502C. Although three such image frames are illustrated in the series of image frames 500 illustrated in FIG. 5, it would be understood by one skilled in the field to which this disclosure pertains that the series of image frames 500 could include more or less image frames. For example, the series of image frames 500 could include thirty image frames.

In some embodiments, the series of image frames 500 may be a time series set of image frames. In this regard, each of the image frames in the series of image frames 500 may be associated with a capture time and the image frames in the series of image frames 500 are ordered in chronological order. For example, the first image frame 502A may be associated with a first capture time, the second image frame 502B may be associated with a second capture time that is later than the first capture time, and the third image frame 502C may be associated with a third capture time that is later than the second capture time. In some embodiments, the first image frame 502A may immediately precede the second image frame 502B and the second image frame 502B may immediately precede the third image frame 502C. Said differently, there may be no other image frames in the series of image frames 500 between the first image frame 502A and the second image frame 502B. Additionally, or alternatively, there may be no other image frames in the series of image frames 500 between the second image frame 502B and the third image frame 502C.

In some embodiments, the series of image frames 500 may include a first portion and a second portion. In some embodiments, the first portion of the series of image frames 500 may be indicative of a gas plume 504. In this regard, for example, the first portion of the series of image frames 500 may be indicative of one or more properties associated with the gas plume 504 that are associated with a non-zero value. For example, the first portion of the series of image frames 500 may be indicative of one or more of a size associated with the gas plume 504, a shape associated with the gas plume 504, a concentration associated with the gas plume 504, and/or the like that are associated with a non-zero value. Said differently, in some embodiments, the first portion of the series of image frames 500 may include image frames in which the gas plume 504 is visible. In some embodiments, the first portion of the series of image frames 500 may include the first image frame 502A and/or the third image frame 502C.

In some embodiments, the second portion of the series of image frames 500 may not be indicative of the gas plume 504. In this regard, for example, the second portion of the series of image frames 500 may be indicative of one or more properties associated with the gas plume 504 that are associated with a zero value. For example, the second portion of the series of image frames 500 may be indicative of one or more of a size of zero associated with the gas plume 504, a concentration of zero associated with the gas plume 504, and/or the like that are associated with a non-zero value. Said differently, in some embodiments, the second portion of the series of image frames 500 may include image frames in which the gas plume 504 is not visible. In some embodiments, the second portion of the series of image frames 500 may include the second image frame 502B.

In some embodiments, the controller component 400 may be configured to generate gas plume impact data. In some embodiments, gas plume impact data may be one or more items of data indicative of a volatility associated with the gas plume 504 as indicated by the image data (e.g., the series of image frames 500). For example, gas plume impact data may be indicative of a volatility associated with a size associated with the gas plume 504, a shape associated with the gas plume 504, a concentration associated with the gas plume 504, and/or the like. In some embodiments, the volatility associated with the gas plume 504 as indicated by the image data may be representative of the amount of change in one or more properties of the gas plume between image frames in the series of image frames 500.

In some embodiments, the controller component 400 may be configured to generate gas plume impact data by applying image data to a gas plume impact model. In some embodiments, the gas plume impact model may be a data entity that describes parameters, hyper-parameters, and/or defined operations of a rules-based, and/or machine learning model (e.g., model including at least one of one or more rule-based layers, one or more layers that depend on trained parameters, coefficients, and/or the like) configured to at least in part generate gas plume impact data. The gas plume impact model may utilize one or more of any type of machine learning and/or rules-based techniques including one or more of supervised learning (e.g., using user feedback), unsupervised learning, semi-supervised learning, reinforcement learning, computer vision techniques, sequence modeling techniques, language processing techniques, and/or neural network techniques.

In some embodiments, applying the image data to the gas plume impact model to generate gas plume impact data may include the gas plume impact model being configured to identify image frames in the second portion of the series of image frames 500 that are immediately preceded by an image frame in the first portion of the series of image frames 500. For example, applying the image data to the gas plume impact model to generate gas plume impact data may include the gas plume impact model being configured to identify the second image frame 502B because the second image frame 502B is in the second portion of the series of image frames 500 and is immediately preceded by the first image frame 502A.

In some embodiments, applying the image data to the gas plume impact model to generate gas plume impact data may include the gas plume impact model being configured to identify image frames in the second portion of the series of image frames 500 that are immediately followed by an image frame in the first portion of the series of image frames 500. For example, applying the image data to the gas plume impact model to generate gas plume impact data may include the gas plume impact model being configured to identify the second image frame 502B because the second image frame 502B is in the second portion of the series of image frames 500 and is immediately followed by the third image frame 502C.

In some embodiments, applying the image data to the gas plume impact model to generate gas plume impact data may include the gas plume impact model being configured to generate zero hit count data. In some embodiments, generating zero hit count data may be based at least in part on determining the number of times in which each image frame in the second portion of the series of image frames 500 is immediately preceded in the series of image frames 500 by an image frame in the first portion of the series of image frames 500. Additionally, or alternatively, generating zero hit count data may be based at least in part on determining the number of times in which each image frame in the second portion of the series of image frames 500 is immediately followed in the series of image frames 500 by an image frame in the first portion of the series of image frames 500. In this regard, for example, zero hit count data may be one or more items of data indicative of and/or associated with a number of times in which each image frame in the second portion of the series of image frames 500 is immediately preceded in the series of image frames 500 by an image frame in the first portion of the series of image frames 500 and/or each image frame in the second portion of the series of image frames 500 is immediately followed in the series of image frames 500 by an image frame in the first portion of the series of image frames 500. For example, for the series of image frames 500 illustrated in FIG. 5, zero hit count data would be indicative of a zero hit count of two because the second image frame 502B is immediately preceded by the first image frame 502A and is immediately followed by the third image frame 502C (e.g., because the second image frame 502B is in the second portion of the series of image frames 500 while the first image frame 502A and the third image frame 502C are in the first portion of the series of image frames 500).

In some embodiments, applying the image data to the gas plume impact model to generate gas plume impact data may include the gas plume impact model being configured to generate gas plume impact data using the zero hit count data. For example, the gas plume impact model may be configured to generate the gas plume impact data using the zero hit count data with equation (1):

$$(1) \ Gas \ Plume \ Impact \ Data = e^{-a*zero \ hit \ count \ data}$$

where a is a hyperparameter (e.g., 2),

In some embodiments, as illustrated by equation (1), as the zero hit count data decreases, the gas plume impact data increases. In some embodiments, the greater the gas plume impact data the less the volatility of the gas plume 504. In this regard, as the zero hit count data decreases the volatility of the gas plume 504 also decreases. Additionally, or alternatively, as illustrated by equation (1), as the zero hit count data increases, the gas plume impact data decreases. In some embodiments, the less the gas plume impact data the greater the volatility of the gas plume 504. In this regard, as the zero hit count data increases the volatility of the gas plume 504 also increases.

In some embodiments, the controller component 400 may be configured to obtain raw gas quantity data. In some embodiments, raw gas quantity data may be one or more items of data indicative of and/or associated with an estimated gas quantity associated with the gas plume 504. In some embodiments, the greater the volatility of the gas plume 504, the less accurate the estimated gas quantity is as indicated by the raw gas quantity data. Said differently, as the volatility of the gas plume 504 increases, the accuracy of the raw gas quantity data decreases. Additionally, or alternatively, as the volatility of the gas plume 504 decreases, the accuracy of the raw gas quantity data increases.

In some embodiments, raw gas quantity data may be obtained from a gas quantity determination machine learning model. In some embodiments, the gas quantity determination machine learning model may be a data entity that describes parameters, hyper-parameters, and/or defined operations of a rules-based, and/or machine learning model (e.g., model including at least one of one or more rule-based layers, one or more layers that depend on trained parameters, coefficients, and/or the like) configured to at least in part generate raw gas quantity data. The gas quantity determination machine learning model may utilize one or more of any type of machine learning and/or rules-based techniques including one or more of supervised learning (e.g., using user feedback), unsupervised learning, semi-supervised learning, reinforcement learning, computer vision techniques, sequence modeling techniques, language processing techniques, and/or neural network techniques.

In some embodiments, the controller component 400 may be configured to generate refined gas quantity data. In some embodiments, refined gas quantity data may be one or more items of data indicative of a gas quantity associated with the gas plume 504. For example, refined gas quantity data may indicate an amount of gas leaked into the environment 100 and/or the asset 106 by a gas leak associated with the gas plume 504. In some embodiments, the controller component 400 may be configured to generate refined gas quantity data based at least in part on gas quantity data and/or gas plume impact data. In some embodiments, the refined gas quantity data may include a first portion and/or a second portion.

In some embodiments, the first portion of the refined gas quantity data may be generated by the controller component 400 being configured to apply gas plume impact data to raw gas quantity data. In some embodiments, applying gas plume impact data to raw gas quantity data may include adjusting the raw gas quantity data by the gas plume impact data. For example, portions of the raw gas quantity data associated with a high volatility, as indicated by a corresponding portion of the gas plume impact data, may be adjusted such that these portions are given a lesser weight in determining the refined gas quantity data (e.g., in determining a gas quantity associated with the gas plume 504). As another example, portions of the raw gas quantity data associated with a low volatility, as indicated by a corresponding portion of the gas plume impact data, may be adjusted such that these portions are given a greater weight in determining the refined gas quantity data (e.g., in determining a gas quantity associated with the gas plume 504). Said differently, an estimated gas quantity associated with the gas plume 504, as indicated by raw gas quantity data may be inaccurate because the volatility of the gas plume 504 is not considered in the generation of the raw gas quantity data. In this regard, for example, applying gas plume impact data to raw gas quantity data to generate refined gas quantity data accounts for volatility of the gas plume 504 in order to obtain an accurate gas quantity associated with the gas plume 504.

In some embodiments, the second portion of the refined gas quantity data may be generated by the controller component 400 being configured to perform an interpolation operation on the first portion of the refined gas quantity data. In some embodiments, the at least one gas detection sensor 310 may be configured to monitor the gas plume 504 as well as one or more other gas plumes in the asset 106 and/or the environment 100. In this regard, for example, there may be time periods in which the gas plume 504 is active but there is no image data captured for these time periods (e.g., because the at least one gas detection sensor 310 is capturing image data of another target area associated with a different gas plume). In some embodiments, the controller component 400 may be configured to perform an interpolation operation on the first portion of the refined gas quantity data to generate refined gas quantity data for time periods in which no image data of the gas plume 504 was captured. Said differently, the second portion of the refined gas quantity data may be indicative of a gas quantity associated with the gas plume 504 for time periods in which the at least one gas detection sensor 310 did not capture any image data of the gas plume 504.

In some embodiments, the controller component 400 may be configured to perform a moving average operation on the refined gas quantity data. In some embodiments, performing a moving average operation may include determining an average gas quantity associated with the gas plume 504 per image frame over a predetermined number of image frames in the series of image frames 500. For example, performing a moving average operation may include determining an average gas quantity associated with the gas plume 504 per image frame over the last three hundred image frames in the series of image frames 500.

In some embodiments, the controller component 400 may be configured to initiate performance of one or more responsive actions. In some embodiments, the controller component 400 may be configured to initiate performance of one or more responsive actions based at least in part on the refined gas quantity data. In some embodiments, initiating performance of one or more responsive actions may include the controller component 400 being configured to trigger an alert associated with the asset 106 and/or the environment 100. For example, an alert may be triggered when the refined gas quantity data indicates that the gas quantity associated with the gas plume 504 is above a threshold. As another example, an alert may be triggered when conditions associated with the environment 100 are causing the raw gas quantity data to be below an accuracy threshold. In this regard, conditions such as wind and/or humidity may be increasing the volatility of the gas plume 504 which is causing the raw gas quantity data to be below an accuracy threshold.

In some embodiments, initiating performance of one or more responsive actions may include the controller component 400 being configured to generate a first interface component 602. In some embodiments, the first interface component 602 may include a raw gas quantity data representation 604. Additionally, or alternatively, the first interface component 602 may include a refined gas quantity data representation 606. Additionally, or alternatively, the first interface component 602 may include a true gas quantity representation 608. Additionally, or alternatively, the first interface component 602 may include an at least one gas detection sensor movement representation 610 (e.g., a representation of when the at least one gas detection sensor 310 moves, such as to monitor different target areas). In some embodiments, initiating performance of one or more responsive actions may include the controller component 400 being configured to cause the first interface component 602 to be rendered to a first interface 600.

In some embodiments, initiating performance of one or more responsive actions may include the controller component 400 being configured to generate a second interface component 702. In some embodiments, the second interface component 702 may include a true gas quantity representation 704. Additionally, or alternatively, the second interface component 702 may include a zero hit count data representation 706 (e.g., each time a zero hit count is identified). In some embodiments, initiating performance of one or more responsive actions may include the controller component 400 being configured to cause the second interface component 702 to be rendered to a second interface 700.

Example Methods

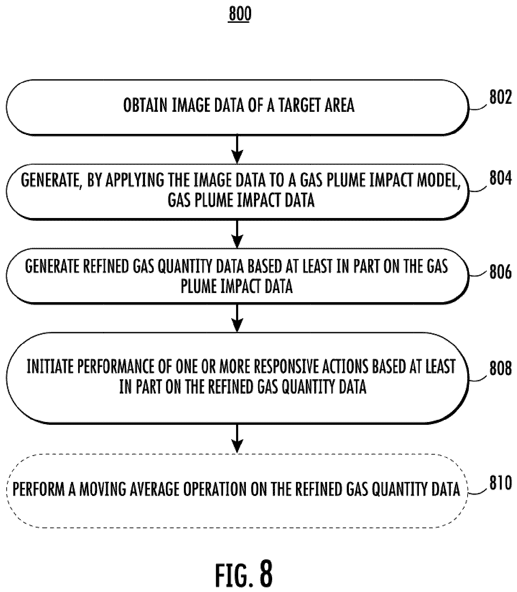

Referring now to FIG. 8, a flowchart diagram illustrating an example method 800 in accordance with various embodiments of the present disclosure is provided. In some examples, the method 800 may be performed by a processing circuitry (for example, but not limited to, an application-specific integrated circuit (ASIC), a central processing unit (CPU)). In some examples, the processing circuitry may be electrically coupled to and/or in electronic communication with other circuitries of the example apparatus, such as, but not limited to, a gas analysis system, the controller component of the gas analysis system, a memory (such as, for example, random access memory (RAM) for storing computer program instructions), and/or a display circuitry (for rendering readings on a display).

As shown in block 802, the method 800 may include obtaining image data of a target area. As described above, in some embodiments, the controller component may be configured to obtain image data from the at least one gas detection sensor. For example, the controller component may be configured to obtain first image data of the first target area from the at least one gas detection sensor. As another example, the controller component may be configured to obtain second image data of the second target area from the at least one gas detection sensor. In some embodiments, the controller component may be configured to cause the at least one gas detection sensor to capture image data (e.g., using the visible gas detection sensor and/or the hyperspectral gas detection sensor).

In some embodiments, image data may include a series of image frames. For example, the series of image frames may include a first image frame. As another example, the series of image frames may include a second image frame. As another example, the series of image frames may include a third image frame. Although three such image frames are illustrated in the series of image frames illustrated in FIG. 5, it would be understood by one skilled in the field to which this disclosure pertains that the series of image frames could include more or less image frames. For example, the series of image frames could include thirty image frames.

In some embodiments, the series of image frames may be a time series set of image frames. In this regard, each of the image frames in the series of image frames may be associated with a capture time and the image frames in the series of image frames are ordered in chronological order. For example, the first image frame may be associated with a first capture time, the second image frame may be associated with a second capture time that is later than the first capture time, and the third image frame may be associated with a third capture time that is later than the second capture time. In some embodiments, the first image frame may immediately precede the second image frame and the second image frame may immediately precede the third image frame. Said differently, there may be no other image frames in the series of image frames between the first image frame and the second image frame. Additionally, or alternatively, there may be no other image frames in the series of image frames between the second image frame and the third image frame.

In some embodiments, the series of image frames may include a first portion and a second portion. In some embodiments, the first portion of the series of image frames may be indicative of a gas plume. In this regard, for example, the first portion of the series of image frames may be indicative of one or more properties associated with the gas plume that are associated with a non-zero value. For example, the first portion of the series of image frames may be indicative of one or more of a size associated with the gas plume, a shape associated with the gas plume, a concentration associated with the gas plume, and/or the like that are associated with a non-zero value. Said differently, in some embodiments, the first portion of the series of image frames may include image frames in which the gas plume is visible. In some embodiments, the first portion of the series of image frames may include the first image frame and/or the third image frame.

In some embodiments, the second portion of the series of image frames may not be indicative of the gas plume. In this regard, for example, the second portion of the series of image frames may be indicative of one or more properties associated with the gas plume that are associated with a zero value. For example, the second portion of the series of image frames may be indicative of one or more of a size of zero associated with the gas plume, a concentration of zero associated with the gas plume, and/or the like that are associated with a non-zero value. Said differently, in some embodiments, the second portion of the series of image frames may include image frames in which the gas plume is not visible. In some embodiments, the second portion of the series of image frames may include the second image frame.

As shown in block 804, the method 800 may include generating, by applying the image data to a gas plume impact model, gas plume impact data. As described above, in some embodiments, gas plume impact data may be one or more items of data indicative of a volatility associated with the gas plume as indicated by the image data (e.g., the series of image frames). For example, gas plume impact data may be indicative of a volatility associated with a size associated with the gas plume, a shape associated with the gas plume, a concentration associated with the gas plume, and/or the like. In some embodiments, the volatility associated with the gas plume as indicated by the image data may be representative of the amount of change in one or more properties of the gas plume between image frames in the series of image frames.

In some embodiments, the controller component may be configured to generate gas plume impact data by applying image data to a gas plume impact model. In some embodiments, the gas plume impact model may be a data entity that describes parameters, hyper-parameters, and/or defined operations of a rules-based, and/or machine learning model (e.g., model including at least one of one or more rule-based layers, one or more layers that depend on trained parameters, coefficients, and/or the like) configured to at least in part generate gas plume impact data. The gas plume impact model may utilize one or more of any type of machine learning and/or rules-based techniques including one or more of supervised learning (e.g., using user feedback), unsupervised learning, semi-supervised learning, reinforcement learning, computer vision techniques, sequence modeling techniques, language processing techniques, and/or neural network techniques.

As shown in block 806, the method 800 may include generating refined gas quantity data based at least in part on the gas plume impact data. As described above, in some embodiments, the controller component may be configured to obtain raw gas quantity data. In some embodiments, raw gas quantity data may be one or more items of data indicative of and/or associated with an estimated gas quantity associated with the gas plume. In some embodiments, the greater the volatility of the gas plume, the less accurate the estimated gas quantity is as indicated by the raw gas quantity data. Said differently, as the volatility of the gas plume increases, the accuracy of the raw gas quantity data decreases. Additionally, or alternatively, as the volatility of the gas plume decreases, the accuracy of the raw gas quantity data increases.

In some embodiments, raw gas quantity data may be obtained from a gas quantity determination machine learning model. In some embodiments, the gas quantity determination machine learning model may be a data entity that describes parameters, hyper-parameters, and/or defined operations of a rules-based, and/or machine learning model (e.g., model including at least one of one or more rule-based layers, one or more layers that depend on trained parameters, coefficients, and/or the like) configured to at least in part generate raw gas quantity data. The gas quantity determination machine learning model may utilize one or more of any type of machine learning and/or rules-based techniques including one or more of supervised learning (e.g., using user feedback), unsupervised learning, semi-supervised learning, reinforcement learning, computer vision techniques, sequence modeling techniques, language processing techniques, and/or neural network techniques.

In some embodiments, the controller component may be configured to generate refined gas quantity data. In some embodiments, refined gas quantity data may be one or more items of data indicative of a gas quantity associated with the gas plume. For example, refined gas quantity data may indicate an amount of gas leaked into the environment and/or the asset by a gas leak associated with the gas plume. In some embodiments, the controller component may be configured to generate refined gas quantity data based at least in part on gas quantity data and/or gas plume impact data. In some embodiments, the refined gas quantity data may include a first portion and/or a second portion.

As shown in block 808, the method 800 may include initiating performance of one or more responsive actions based at least in part on the refined gas quantity data. As described above, in some embodiments, the controller component may be configured to initiate performance of one or more responsive actions based at least in part on the refined gas quantity data. In some embodiments, initiating performance of one or more responsive actions may include the controller component being configured to trigger an alert associated with the asset and/or the environment. For example, an alert may be triggered when the refined gas quantity data indicates that the gas quantity associated with the gas plume is above a threshold. As another example, an alert may be triggered when conditions associated with the environment are causing the raw gas quantity data to be below an accuracy threshold. In this regard, conditions such as wind and/or humidity may be increasing the volatility of the gas plume which is causing the raw gas quantity data to be below an accuracy threshold.

In some embodiments, initiating performance of one or more responsive actions may include the controller component being configured to generate a first interface component. In some embodiments, the first interface component may include a raw gas quantity data representation. Additionally, or alternatively, the first interface component may include a refined gas quantity data representation. Additionally, or alternatively, the first interface component may include a true gas quantity representation. Additionally, or alternatively, the first interface component may include an at least one gas detection sensor movement representation (e.g., a representation of when the at least one gas detection sensor moves, such as to monitor different target areas). In some embodiments, initiating performance of one or more responsive actions may include the controller component being configured to cause the first interface component to be rendered to a first interface.

In some embodiments, initiating performance of one or more responsive actions may include the controller component being configured to generate a second interface component. In some embodiments, the second interface component may include a true gas quantity representation. Additionally, or alternatively, the second interface component may include a zero hit count data representation (e.g., each time a zero hit count is identified). In some embodiments, initiating performance of one or more responsive actions may include the controller component being configured to cause the second interface component to be rendered to a second interface.

As shown in block 810, the method 800 may optionally include performing a moving average operation on the refined gas quantity data. As described above, in some embodiments, performing a moving average operation may include determining an average gas quantity associated with the gas plume per image frame over a predetermined number of image frames in the series of image frames. For example, performing a moving average operation may include determining an average gas quantity associated with the gas plume per image frame over the last three hundred image frames in the series of image frames.

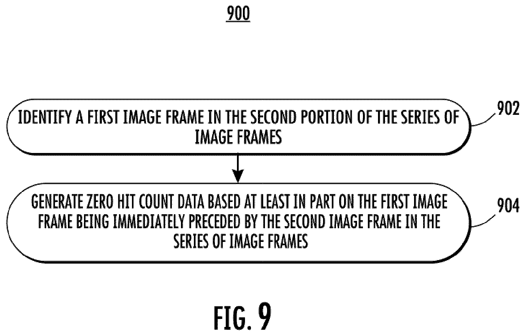

Referring now to FIG. 9, a flowchart diagram illustrating an example method 900 in accordance with various embodiments of the present disclosure is provided. In some embodiments, the method 900 may include one or more operations for applying image data to the gas plume impact model to generate gas plume impact data. For example, the method 900 may include one or more operations for generating zero hit count data based at least in part on the first image frame being immediately preceded by the second image frame in the series of image frames. In some examples, the method 900 may be performed by a processing circuitry (for example, but not limited to, an application-specific integrated circuit (ASIC), a central processing unit (CPU)). In some examples, the processing circuitry may be electrically coupled to and/or in electronic communication with other circuitries of the example apparatus, such as, but not limited to, a gas analysis system, the controller component of the gas analysis system, a memory (such as, for example, random access memory (RAM) for storing computer program instructions), and/or a display circuitry (for rendering readings on a display).

As shown in block 902, the method 900 may include identifying a first image frame in the second portion of the series of image frames. As described above, in some embodiments, applying the image data to the gas plume impact model to generate gas plume impact data may include the gas plume impact model being configured to identify image frames in the second portion of the series of image frames that are immediately preceded by an image frame in the first portion of the series of image frames. For example, applying the image data to the gas plume impact model to generate gas plume impact data may include the gas plume impact model being configured to identify the second image frame because the second image frame is in the second portion of the series of image frames and is immediately preceded by the first image frame.

As shown in block 904, the method 900 may include generating zero hit count data based at least in part on the first image frame being immediately preceded by the second image frame in the series of image frames. As described above, in some embodiments, generating zero hit count data may be based at least in part on determining the number of times in which each image frame in the second portion of the series of image frames is immediately preceded in the series of image frames by an image frame in the first portion of the series of image frames. Additionally, or alternatively, generating zero hit count data may be based at least in part on determining the number of times in which each image frame in the second portion of the series of image frames is immediately followed in the series of image frames by an image frame in the first portion of the series of image frames. In this regard, for example, zero hit count data may be one or more items of data indicative of and/or associated with a number of times in which each image frame in the second portion of the series of image frames is immediately preceded in the series of image frames by an image frame in the first portion of the series of image frames and/or each image frame in the second portion of the series of image frames is immediately followed in the series of image frames by an image frame in the first portion of the series of image frames. For example, for the series of image frames illustrated in FIG. 5, zero hit count data would be indicative of a zero hit count of two because the second image frame is immediately preceded by the first image frame and is immediately followed by the third image frame (e.g., because the second image frame is in the second portion of the series of image frames while the first image frame and the third image frame are in the first portion of the series of image frames).

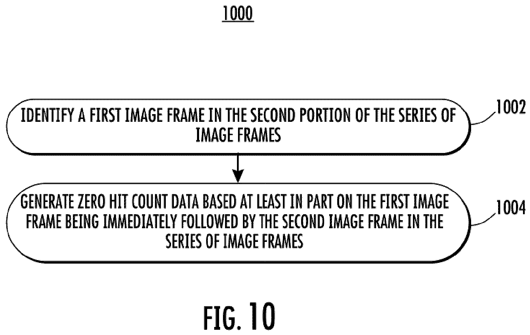

Referring now to FIG. 10, a flowchart diagram illustrating an example method 1000 in accordance with various embodiments of the present disclosure is provided. In some embodiments, the method 1000 may include one or more operations for applying image data to the gas plume impact model to generate gas plume impact data. For example, the method 1000 may include one or more operations for generating zero hit count data based at least in part on the first image frame being immediately followed by the second image frame in the series of image frames. In some examples, the method 1000 may be performed by a processing circuitry (for example, but not limited to, an application-specific integrated circuit (ASIC), a central processing unit (CPU)). In some examples, the processing circuitry may be electrically coupled to and/or in electronic communication with other circuitries of the example apparatus, such as, but not limited to, a gas analysis system, the controller component of the gas analysis system, a memory (such as, for example, random access memory (RAM) for storing computer program instructions), and/or a display circuitry (for rendering readings on a display).

As shown in block 1002, the method 1000 may include identifying a first image frame in the second portion of the series of image frames. As described above, in some embodiments, applying the image data to the gas plume impact model to generate gas plume impact data may include the gas plume impact model being configured to identify image frames in the second portion of the series of image frames that are immediately followed by an image frame in the first portion of the series of image frames. For example, applying the image data to the gas plume impact model to generate gas plume impact data may include the gas plume impact model being configured to identify the second image frame because the second image frame is in the second portion of the series of image frames and is immediately followed by the third image frame.

As shown in block 1004, the method 1000 may include generating zero hit count data based at least in part on the first image frame being immediately followed by the second image frame in the series of image frames. As described above, in some embodiments, generating zero hit count data may be based at least in part on determining the number of times in which each image frame in the second portion of the series of image frames is immediately preceded in the series of image frames by an image frame in the first portion of the series of image frames. Additionally, or alternatively, generating zero hit count data may be based at least in part on determining the number of times in which each image frame in the second portion of the series of image frames is immediately followed in the series of image frames by an image frame in the first portion of the series of image frames. In this regard, for example, zero hit count data may be one or more items of data indicative of and/or associated with a number of times in which each image frame in the second portion of the series of image frames is immediately preceded in the series of image frames by an image frame in the first portion of the series of image frames and/or each image frame in the second portion of the series of image frames is immediately followed in the series of image frames by an image frame in the first portion of the series of image frames. For example, for the series of image frames illustrated in FIG. 5, zero hit count data would be indicative of a zero hit count of two because the second image frame is immediately preceded by the first image frame and is immediately followed by the third image frame (e.g., because the second image frame is in the second portion of the series of image frames while the first image frame and the third image frame are in the first portion of the series of image frames).

In some embodiments, applying the image data to the gas plume impact model to generate gas plume impact data may include the gas plume impact model being configured to generate gas plume impact data using the zero hit count data. For example, the gas plume impact model may be configured to generate the gas plume impact data using the zero hit count data with equation (2):

$$(2)\ Gas\ Plume\ Impact\ Data = e^{-a*zero\ hit\ count\ data}$$

where a is a hyperparameter (e.g., 2),

In some embodiments, as illustrated by equation (2), as the zero hit count data decreases, the gas plume impact data increases. In some embodiments, the greater the gas plume impact data the less the volatility of the gas plume. In this regard, as the zero hit count data decreases the volatility of the gas plume also decreases. Additionally, or alternatively, as illustrated by equation (2), as the zero hit count data increases, the gas plume impact data decreases. In some embodiments, the less the gas plume impact data the greater the volatility of the gas plume. In this regard, as the zero hit count data increases the volatility of the gas plume also increases.

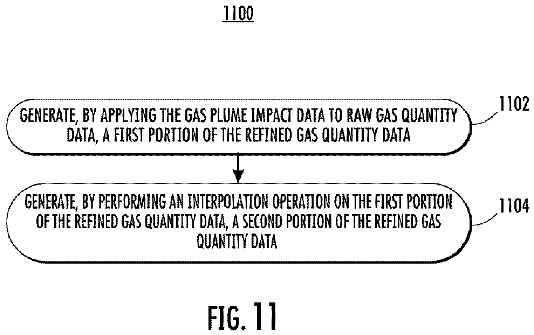

Referring now to FIG. 11, a flowchart diagram illustrating an example method 1100 in accordance with various embodiments of the present disclosure is provided. In some embodiments, the method 1100 may include one or more operations for applying image data to the gas plume impact model to generate gas plume impact data. For example, the method 1100 may include one or more operations for generating zero hit count data based at least in part on the first image frame being immediately followed by the second image frame in the series of image frames. In some examples, the method 1100 may be performed by a processing circuitry (for example, but not limited to, an application-specific integrated circuit (ASIC), a central processing unit (CPU)). In some examples, the processing circuitry may be electrically coupled to and/or in electronic communication with other circuitries of the example apparatus, such as, but not limited to, a gas analysis system, the controller component of the gas analysis system, a memory (such as, for example, random access memory (RAM) for storing computer program instructions), and/or a display circuitry (for rendering readings on a display).

As shown in block 1102, the method 1100 may include generating, by applying the gas plume impact data to raw gas quantity data, a first portion of the refined gas quantity data. As described above, in some embodiments, the first portion of the refined gas quantity data may be generated by the controller component being configured to apply gas plume impact data to raw gas quantity data. In some embodiments, applying gas plume impact data to raw gas quantity data may include adjusting the raw gas quantity data by the gas plume impact data. For example, portions of the raw gas quantity data associated with a high volatility, as indicated by a corresponding portion of the gas plume impact data, may be adjusted such that these portions are given a lesser weight in determining the refined gas quantity data (e.g., in determining a gas quantity associated with the gas plume). As another example, portions of the raw gas quantity data associated with a low volatility, as indicated by a corresponding portion of the gas plume impact data, may be adjusted such that these portions are given a greater weight in determining the refined gas quantity data (e.g., in determining a gas quantity associated with the gas plume). Said differently, an estimated gas quantity associated with the gas plume, as indicated by raw gas quantity data may be inaccurate because the volatility of the gas plume is not considered in the generation of the raw gas quantity data. In this regard, for example, applying gas plume impact data to raw gas quantity data to generate refined gas quantity data accounts for volatility of the gas plume in order to obtain an accurate gas quantity associated with the gas plume.

As shown in block 1104, the method 1100 may include generating, by performing an interpolation operation on the first portion of the refined gas quantity data, a second portion of the refined gas quantity data. As described above, in some embodiments, the second portion of the refined gas quantity data may be generated by the controller component being configured to perform an interpolation operation on the first portion of the refined gas quantity data. In some embodiments, the at least one gas detection sensor may be configured to monitor the gas plume as well as one or more other gas plumes in the asset and/or the environment. In this regard, for example, there may be time periods in which the gas plume is active but there is no image data captured for these time periods (e.g., because the at least one gas detection sensor is capturing image data of another target area associated with a different gas plume). In some embodiments, the controller component may be configured to perform an interpolation operation on the first portion of the refined gas quantity data to generate refined gas quantity data for time periods in which no image data of the gas plume was captured. Said differently, the second portion of the refined gas quantity data may be indicative of a gas quantity associated with the gas plume for time periods in which the at least one gas detection sensor did not capture any image data of the gas plume.

Operations and/or functions of the present disclosure have been described herein, such as in flowcharts. As will be appreciated, computer program instructions may be loaded onto a computer or other programmable apparatus (e.g., hardware) to produce a machine, such that the resulting computer or other programmable apparatus implements the operations and/or functions described in the flowchart blocks herein. These computer program instructions may also be stored in a computer-readable memory that may direct a computer, processor, or other programmable apparatus to operate and/or function in a particular manner, such that the instructions stored in the computer-readable memory produce an article of manufacture, the execution of which implements the operations and/or functions described in the flowchart blocks. The computer program instructions may also be loaded onto a computer, processor, or other programmable apparatus to cause a series of operations to be performed on the computer, processor, or other programmable apparatus to produce a computer-implemented process such that the instructions executed on the computer, processor, or other programmable apparatus provide operations for implementing the functions and/or operations specified in the flowchart blocks. The flowchart blocks support combinations of means for performing the specified operations and/or functions and combinations of operations and/or functions for performing the specified operations and/or functions. It will be understood that one or more blocks of the flowcharts, and combinations of blocks in the flowcharts, can be implemented by special purpose hardware-based computer systems which perform the specified operations and/or functions, or combinations of special purpose hardware with computer instructions.

While operations and/or functions are illustrated in the drawings in a particular order, this should not be understood as requiring that such operations and/or functions be performed in the particular order shown or in sequential order, or that all illustrated operations be performed, to achieve desirable results. In certain circumstances, operations and/or functions in alternative ordering may be advantageous. In some cases, the actions recited in the claims may be performed in a different order and still achieve desirable results. Thus, while particular embodiments of the subject matter have been described, other embodiments are within the scope of the following claims.

While this specification contains many specific embodiment and implementation details, these should not be construed as limitations on the scope of any disclosures or of what may be claimed, but rather as descriptions of features specific to particular embodiments of particular disclosures. Certain features that are described herein in the context of separate embodiments can also be implemented in combination in a single embodiment. Conversely, various features that are described in the context of a single embodiment can also be implemented in multiple embodiments separately or in any suitable subcombination. Moreover, although features may be described above as acting in certain combinations and even initially claimed as such, one or more features from a claimed combination can in some cases be excised from the combination, and the claimed combination may be directed to a subcombination or variation of a subcombination.

Similarly, while operations are illustrated in the drawings in a particular order, this should not be understood as requiring that such operations be performed in the particular order shown or in sequential order, or that all illustrated operations be performed, to achieve desirable results. In certain circumstances, operations in alternative ordering may be advantageous. In some cases, the actions recited in the claims may be performed in a different order and still achieve desirable results.

**Claims**

1. A gas analysis system comprising:

   at least one gas detection sensor; and
   a controller component, wherein the controller component is configured to:

   obtain image data of a target area, wherein the image data comprises a series of image frames, wherein a first portion of the series of image frames are indicative of a gas plume;
   generate, by applying the image data to a gas plume impact model, gas plume impact data;
   generate refined gas quantity data based at least in part on the gas plume impact data; and
   initiate performance of one or more responsive actions based at least in part on the refined gas quantity data.

2. The gas analysis system of claim 1, wherein the target area is associated with an asset.

3. The gas analysis system of claim 2, wherein the asset is a processing plant.

4. The gas analysis system of claim 1, wherein the controller component is further configured to:
   perform a moving average operation on the refined gas quantity data.

5. The gas analysis system of claim 1, wherein a second portion of the series of image frames are not indicative of the gas plume.

6. The gas analysis system of claim 5, wherein applying the image data to the gas plume impact model to generate gas plume impact data comprises the gas plume impact model being configured to:

   identify a first image frame in the second portion of the series of image frames, wherein the first image frame is immediately preceded in the series of image frames by a second image frame in the first portion of the series of image frames; and
   generate zero hit count data based at least in part on the first image frame being immediately preceded by the second image frame in the series of image frames.

7. The gas analysis system of claim 5, wherein applying the image data to the gas plume impact model to generate gas plume impact data comprises the gas plume impact model being configured to:

   identify a first image frame in the second portion of the series of image frames, wherein the first image frame is immediately followed in the series of image frames by a second image frame in the first portion of the series of image frames; and
   generate zero hit count data based at least in part on the first image frame being immediately followed by the second image frame in the series of image frames.

8. The gas analysis system of claim 1, wherein generating the refined gas quantity data comprises:
   generating, by applying the gas plume impact data to raw gas quantity data, a first portion of the refined gas quantity data.

9. The gas analysis system of claim 8, wherein the raw gas quantity data is generated by applying the image data to a gas quantity determination machine learning model.

10. The gas analysis system of claim 8, wherein generating the refined gas quantity data comprises:
    generating, by performing an interpolation operation on the first portion of the refined gas quantity data, a second portion of the refined gas quantity data.

11. A method comprising:

obtaining image data of a target area, wherein the image data comprises a series of image frames, wherein a first portion of the series of image frames are indicative of a gas plume;

generating, by applying the image data to a gas plume impact model, gas plume impact data;

generating refined gas quantity data based at least in part on the gas plume impact data; and

initiating performance of one or more responsive actions based at least in part on the refined gas quantity data.

12. The method of claim 11, wherein the target area is associated with an asset, wherein the asset is a processing plant.

13. The method of claim 11, further comprising:

performing a moving average operation on the refined gas quantity data.

14. The method of claim 11, wherein a second portion of the series of image frames are not indicative of the gas plume.

15. A computer program product comprising at least one non-transitory computer-readable storage medium having computer program code stored thereon that, in execution with at least one processor, configures the computer program product for:

obtaining image data of a target area, wherein the image data comprises a series of image frames, wherein a first portion of the series of image frames are indicative of a gas plume;

generating, by applying the image data to a gas plume impact model, gas plume impact data;

generating refined gas quantity data based at least in part on the gas plume impact data; and

initiating performance of one or more responsive actions based at least in part on the refined gas quantity data.

FIG. 1

FIG. 2

FIG. 3

FIG. 4

FIG. 5

FIG. 6

FIG. 7

800

```
┌─────────────────────────────────────────────────────┐
│         OBTAIN IMAGE DATA OF A TARGET AREA            │──802
└─────────────────────────────────────────────────────┘
                          │
                          ▼
┌─────────────────────────────────────────────────────┐
│ GENERATE, BY APPLYING THE IMAGE DATA TO A GAS PLUME   │──804
│     IMPACT MODEL, GAS PLUME IMPACT DATA               │
└─────────────────────────────────────────────────────┘
                          │
                          ▼
┌─────────────────────────────────────────────────────┐
│ GENERATE REFINED GAS QUANTITY DATA BASED AT LEAST IN  │──806
│      PART ON THE GAS PLUME IMPACT DATA                │
└─────────────────────────────────────────────────────┘
                          │
                          ▼
┌─────────────────────────────────────────────────────┐
│ INITIATE PERFORMANCE OF ONE OR MORE RESPONSIVE        │──808
│ ACTIONS BASED AT LEAST IN PART ON THE REFINED GAS     │
│ QUANTITY DATA                                         │
└─────────────────────────────────────────────────────┘
                          │
                          ▼
┌ ─ ─ ─ ─ ─ ─ ─ ─ ─ ─ ─ ─ ─ ─ ─ ─ ─ ─ ─ ─ ─ ─ ─ ─ ─ ─ ┐
  PERFORM A MOVING AVERAGE OPERATION ON THE REFINED      ──810
│ GAS QUANTITY DATA                                     │
└ ─ ─ ─ ─ ─ ─ ─ ─ ─ ─ ─ ─ ─ ─ ─ ─ ─ ─ ─ ─ ─ ─ ─ ─ ─ ─ ┘
```

FIG. 8

900

IDENTIFY A FIRST IMAGE FRAME IN THE SECOND PORTION OF THE SERIES OF
IMAGE FRAMES
— 902

GENERATE ZERO HIT COUNT DATA BASED AT LEAST IN PART ON THE FIRST IMAGE
FRAME BEING IMMEDIATELY PRECEDED BY THE SECOND IMAGE FRAME IN THE
SERIES OF IMAGE FRAMES
— 904

FIG. 9

1000

IDENTIFY A FIRST IMAGE FRAME IN THE SECOND PORTION OF THE SERIES OF IMAGE FRAMES ⟩⌐1002

GENERATE ZERO HIT COUNT DATA BASED AT LEAST IN PART ON THE FIRST IMAGE FRAME BEING IMMEDIATELY FOLLOWED BY THE SECOND IMAGE FRAME IN THE SERIES OF IMAGE FRAMES ⟩⌐1004

FIG. 10

1100

GENERATE, BY APPLYING THE GAS PLUME IMPACT DATA TO RAW GAS QUANTITY DATA, A FIRST PORTION OF THE REFINED GAS QUANTITY DATA — 1102

GENERATE, BY PERFORMING AN INTERPOLATION OPERATION ON THE FIRST PORTION OF THE REFINED GAS QUANTITY DATA, A SECOND PORTION OF THE REFINED GAS QUANTITY DATA — 1104

FIG. 11

Europäisches
Patentamt

European
Patent Office

Office européen
des brevets

# EUROPEAN SEARCH REPORT

**Application Number**

EP 25 16 7796

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2022/398684 A1 (WHITING OZGE CAN [US] ET AL) 15 December 2022 (2022-12-15) * abstract * * paragraph [0029] - paragraph [0073] * ----- | 1-15 | INV. G01N33/00 |
| A | US 2019/162657 A1 (WANG LEIMING [US] ET AL) 30 May 2019 (2019-05-30) * the whole document * ----- | 1-15 | |

**TECHNICAL FIELDS SEARCHED (IPC)**

G01N

The present search report has been drawn up for all claims

| Place of search | Date of completion of the search | Examiner |
|---|---|---|
| The Hague | 29 July 2025 | Appeltant, Lennert |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

 .................................................................
& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 25 16 7796

This annex lists the patent family members relating to the patent documents cited in the above-mentioned European search report.
The members are as contained in the European Patent Office EDP file on
The European Patent Office is in no way liable for these particulars which are merely given for the purpose of information.

29-07-2025

| Patent document cited in search report | Publication date | Patent family member(s) | | Publication date |
|---|---|---|---|---|
| US 2022398684 A1 | 15-12-2022 | AU | 2020253570 A1 | 04-11-2021 |
| | | CA | 3134710 A1 | 08-10-2020 |
| | | EP | 3948201 A1 | 09-02-2022 |
| | | US | 2020320659 A1 | 08-10-2020 |
| | | US | 2022398684 A1 | 15-12-2022 |
| | | WO | 2020206407 A2 | 08-10-2020 |
| | | WO | 2020206408 A1 | 08-10-2020 |
| US 2019162657 A1 | 30-05-2019 | EP | 3462413 A1 | 03-04-2019 |
| | | JP | 7042196 B2 | 25-03-2022 |
| | | JP | 2019066465 A | 25-04-2019 |
| | | US | 10234380 B1 | 19-03-2019 |
| | | US | 2019162657 A1 | 30-05-2019 |

EPO FORM P0459

For more details about this annex : see Official Journal of the European Patent Office, No. 12/82